# EUROPEAN PATENT APPLICATION

(11) **EP 2 684 522 A1**
(43) Date of publication of application: **15.01.2014**
(21) Application number: 13176382.3
(22) Date of filing: 12.07.2013
(51) Int. Cl.: A61B 6/00

(54) **X-ray imaging apparatus, remote controller for X-ray imaging apparatus, and control method for X-ray imaging apparatus**

(30) Priority: 12.07.2012 US 201261670772 P; 26.03.2013 KR 20130031970
(71) Applicant: Samsung Electronics Co., Ltd, Gyeonggi-do 443-742 (KR)
(72) Inventor: Sung, Young Hun, Hwaseong-si Gyeonggi-do (KR); Kang, Dong Goo, Hwaseong-si Gyeonggi-do (KR); Jang, Kwan Eun, Seoul (KR)
(74) Representative: Grünecker, Kinkeldey, Stockmair & Schwanhäusser

(57) **Abstract**

Disclosed are an X-ray imaging apparatus, a remote controller for the X-ray imaging apparatus, and a control method for the X-ray imaging apparatus. The X-ray imaging apparatus includes a movable X-ray emitter to emit X-rays to a subject, a movable X-ray detector to detect X-rays emitted from the X-ray emitter and change the X-rays into electric signals, an input unit to receive target position information regarding at least one of the X-ray emitter and the X-ray detector, and a controller to control movements of the X-ray emitter and the X-ray detector according to the input target position information.

## Description

### 1. Field

Embodiments of the present invention relate to an X-ray imaging apparatus, a remote controller for the X-ray imaging apparatus, and a control method for the X-ray imaging apparatus.

### 2. Description of the Related Art

An X-ray imaging apparatus is an imaging system that acquires an image inside a subject by emitting X-rays (also referred to as Roentgen rays) to the subject, such as a human body, luggage, etc., and detecting tissues or structures inside the subject, or objects inside the subject, such as a human body, luggage, etc., and that allows a user, for example, a doctor or a diagnostician to visually check tissues, structures, and objects inside the subject.

Such an X-ray imaging apparatus is based on transmission or absorption of X-rays directed to the subject according to properties, e.g., density of a material at which X-rays arrived.

The principle of a general X-ray imaging apparatus is as follows.

If X-rays are emitted to a subject, such as a human body, X-rays having passed through or directed around the subject are received. Thereafter, the received X-rays are changed into electric signals, and an X-ray image is read out from the electric signals. The acquired X-ray image shows tissues, structures or materials inside the subject.

Accordingly, the X-ray imaging apparatus may be used to detect any diseases or other abnormalities of a human body, or to observe internal structures of objects or components, and may also be used to scan luggage in the airport, etc.

Examples of the X-ray imaging apparatus may include a Digital Radiography (DR), Computed Tomography (CT) or Full Field Digital Mammography (FFDM) apparatus.

### SUMMARY

It is an aspect of the present invention to provide an X-ray imaging apparatus, a remote controller for the X-ray imaging apparatus, and a control method for the X-ray imaging apparatus, in which movements of both an X-ray emitter (X-ray source) that emits X-rays to a subject and an X-ray detector that receives the emitted X-rays may be controlled.

It is another aspect of the present invention to ensure transmission of information regarding target positions or control instructions that command movement to particular positions to both an X-ray emitter and an X-ray detector.

It is another aspect of the present invention to provide a device to remotely control movements of both an X-ray emitter and an X-ray detector.

It is another aspect of the present invention to determine an optimal position where an image of a subject is captured during X-ray imaging using an X-ray emitter and an X-ray detector of an X-ray imaging apparatus.

It is a further aspect of the present invention to acquire an X-ray image at an accurate view via implementation of X-ray imaging at an optimal position.

Additional aspects of the invention will be set forth in part in the description which follows and, in part, will be obvious from the description, or may be learned by practice of the invention.

In accordance with one aspect of the invention, an X-ray imaging apparatus includes a movable X-ray emitter to emit X-rays to a subject, a movable X-ray detector to detect X-rays emitted from the X-ray emitter, an input unit to receive target position information regarding at least one of the X-ray emitter and the X-ray detector, and a controller to control movements of the X-ray emitter and the X-ray detector according to the input target position information.

The input unit may receive target position information regarding the X-ray emitter and target position information regarding the X-ray detector respectively, and the controller may control movements of the X-ray emitter and the X-ray detector according to the respective input target position information.

The controller may receive target position information from a remote controller.

In accordance with another aspect of the invention, a control method for an X-ray imaging apparatus includes receiving at least one of target position information regarding an X-ray emitter and target position information regarding an X-ray detector, and moving the X-ray emitter and the X-ray detector according to the target position information.

The reception of the target position information may include receiving the target position information regarding the X-ray emitter and the target position information regarding the X-ray detector respectively, and the movement of the X-ray emitter and the X-ray detector may include moving the X-ray emitter and the X-ray detector according to the respective input target position information.

### BRIEF DESCRIPTION OF THE DRAWINGS

These and/or other aspects of the invention will become apparent and more readily appreciated from the following description of the embodiments, taken in conjunction with the accompanying drawings of which:
FIG. 1A is a view illustrating one embodiment of a system to control an X-ray emitter and an X-ray detector in an X-ray imaging apparatus;
FIG. 1 B is a view illustrating a configuration of an X-ray imaging apparatus according to an embodiment;
FIG. 2 is a front view illustrating an embodiment of an X-ray imaging apparatus;
FIG. 3 is a perspective view illustrating an embodiment of an X-ray emission module;
FIG. 4 is a view illustrating an embodiment of an X-ray emitter;
FIG. 5 is a view illustrating an embodiment of an X-ray detection module;
FIG. 6 is a view illustrating an embodiment of an X-ray detection module;
FIG. 7 is a view illustrating another embodiment of an X-ray detection module;
FIG. 8 is a block diagram illustrating an embodiment of a controller;
FIG. 9A is a view illustrating another embodiment of a system to control an X-ray emitter and an X-ray detector in an X-ray imaging apparatus;
FIG. 9B is a view illustrating a configuration of an X-ray imaging apparatus according to another embodiment;
FIGS. 10 to 12 are views explaining an embodiment of a remote controller and an X-ray imaging apparatus;
FIGS. 13 and 14 are respectively a block diagram and a side view explaining an embodiment of an X-ray imaging apparatus equipped with a position information collector; and
FIGS. 15 and 16 are flowcharts illustrating a control method for an X-ray imaging apparatus.

### DETAILED DESCRIPTION

Reference will now be made in detail to the embodiments of the present invention, examples of which are illustrated in the accompanying drawings, wherein like reference numerals refer to like elements throughout.

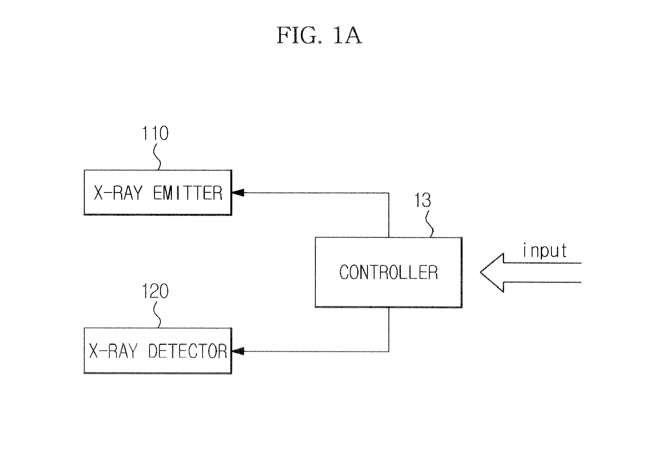

FIG. 1A is a view illustrating an embodiment of a system to control an X-ray emitter and an X-ray detector in an X-ray imaging apparatus;

As exemplarily illustrated in FIG. 1A, according to an embodiment, the X-ray imaging apparatus may include an X-ray emitter 110, an X-ray detector 120, and a controller 13. Here, to control movements of the X-ray emitter 110 and the X-ray detector 120, the controller 13 transmits information or instructions to each of the X-ray emitter 110 and the X-ray detector 120, and controls the X-ray emitter 110 and the X-ray detector 120 so as to be moved based on the transmitted information or instructions. After moved, the X-ray emitter 110 emits X-rays to a subject. As necessary, the controller 13 may receive external data or instructions, and transmit the received data or instructions to the X-ray emitter 110 and the X-ray detector 120. In this case, the controller 13 may generate novel data or instructions based on the received data or instructions, and thereafter transmit the generated novel data or instructions to the X-ray emitter 110 and the X-ray detector 120.

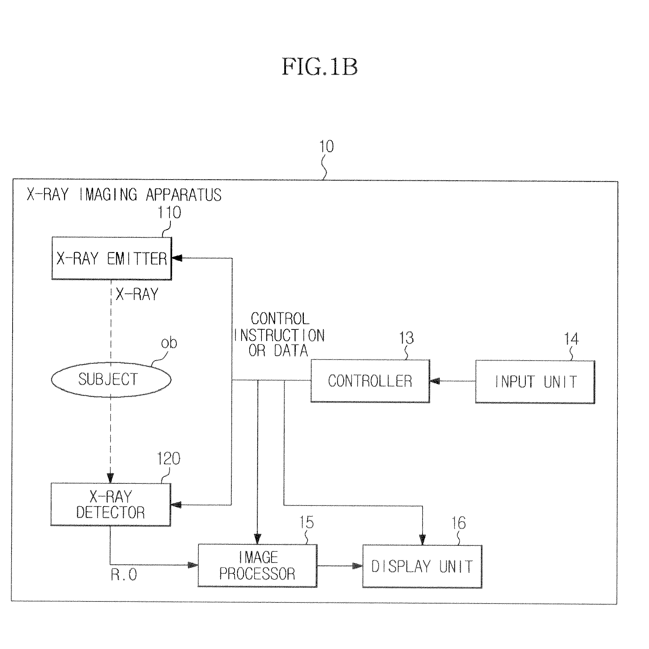

FIG. 1 B is a view illustrating a configuration of the X-ray imaging apparatus according to the embodiment.

Explaining the embodiment of the X-ray imaging apparatus illustrated in FIG. 1A in more detail, as exemplarily illustrated in FIG. 1 B, the X-ray imaging apparatus includes the X-ray detector 110 that emits X-rays to the subject, the X-ray detector 120 that receives the emitted X-rays and changes the received X-rays into electric signals, and the controller 13. The X-ray emitter 110 and the X-ray detector 120 may be designed so as to be movable to a plurality of positions and to emit X-rays or receive the emitted X-rays at the moved positions. In addition, the X-ray imaging apparatus may include at least one of an input unit 14 that allows a user to input instructions for control of the X-ray imaging apparatus, an image processor 15 that generates an X-ray image using the electric signals acquired by the X-ray detector 120, and a display unit 16 that displays the X-ray image generated by the image processor 15 to a user. As necessary, the X-ray imaging apparatus may include all of the input unit 14, the image processor 15 and the display unit 16.

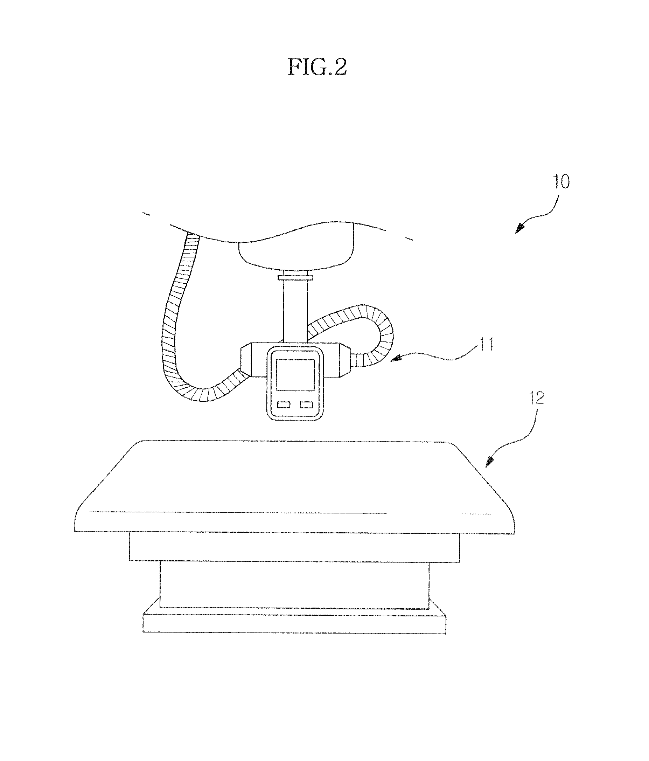

FIG. 2 is a front view illustrating an embodiment of an X-ray imaging apparatus.

One example of the aforementioned X-ray imaging apparatus may include a Digital Radiography (DR) apparatus as exemplarily illustrated in FIG. 2. The DR apparatus may include an upper X-ray emission module 11 that is movable to a predetermined position, and a lower X-ray detection module 12 in the form of a table on which a subject is placed.

Although the following description is based on the DR apparatus according to one embodiment of the X-ray imaging apparatus, the embodiment of the X-ray imaging apparatus is not limited thereto. The embodiment of the X-ray imaging apparatus may be applied to various other apparatuses, such as a CT or FFDM apparatus.

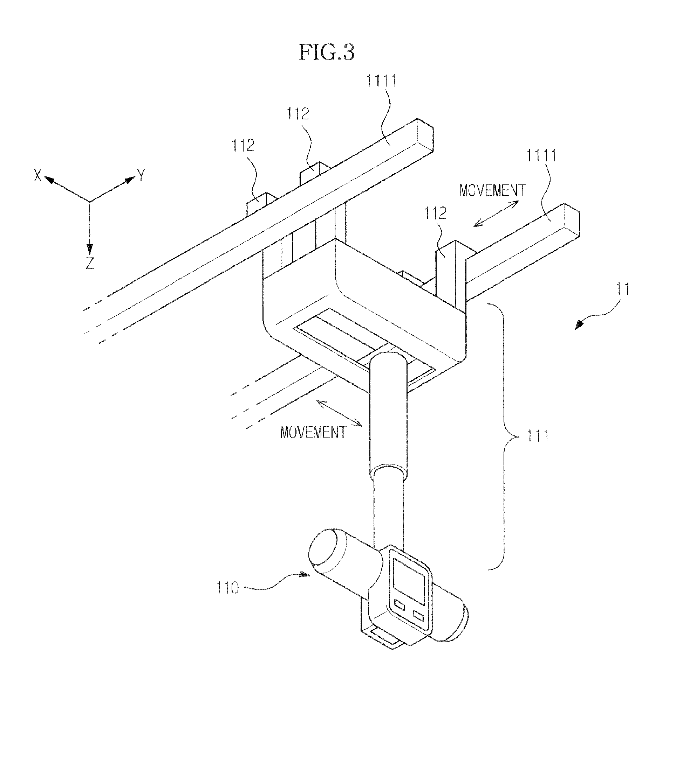

FIG. 3 is a perspective view illustrating an embodiment of the X-ray emission module.

The X-ray emission module 11 is movably designed to emit X-rays to a subject *ob* at a plurality of positions. As exemplarily illustrated in FIG. 3, the X-ray emission module 11 may include a mover 111. The mover 111 may include, for example, a rail 1111 and a connector 112 that travels along the rail 1111.

The connector 112, for example, may include canisters that are guided along the rail 1111 while coming into contact with the rail 1111. When the connector 112 travels along the rail 1111, another component of the X-ray emission module 111 connected to the rail 1111 through the connector 112, for example, the X-ray emitter 110, is moved to a predetermined position via movement of the connector 112.

The rail 1111, as exemplarily illustrated in FIG. 3, may be installed in a line in an upper region of the X-ray emission module 11. For example, the X-ray emitter 110 of the X-ray emission module 11 may be moved in at least one direction along the rail 1111 that is installed in a line. Additionally, a plurality of rails may be installed in a plurality of directions, which may allow, for example, the X-ray emitter 110 of the X-ray emission module 11 to be moved in several directions, for example, in the X-axis and the Y-axis.

Alternatively, the X-ray emission module 11 may include, for example, a robot arm instead of the rail 1111. In this case, for example, the X-ray emitter may be designed so as to be connected to the robot arm and to be moved to a predetermined position via driving of the robot arm. Additionally, the X-ray emission module 11 may be designed so as to move, for example, the X-ray emitter 110 to a predetermined position via operation of a pneumatic or hydraulic cylinder connected to the X-ray emitter 110. Other devices to move a position of a designated object may be applied to the X-ray emission module 11 so as to move the X-ray emission module 11 within a predetermined range.

The X-ray emitter 110 to emit X-rays may be arranged in a lower region of the X-ray emission module 11. The X-ray emitter 110 may be installed at several positions of the X-ray emission module 11 including, for example, a lower end of the X-ray emission module.

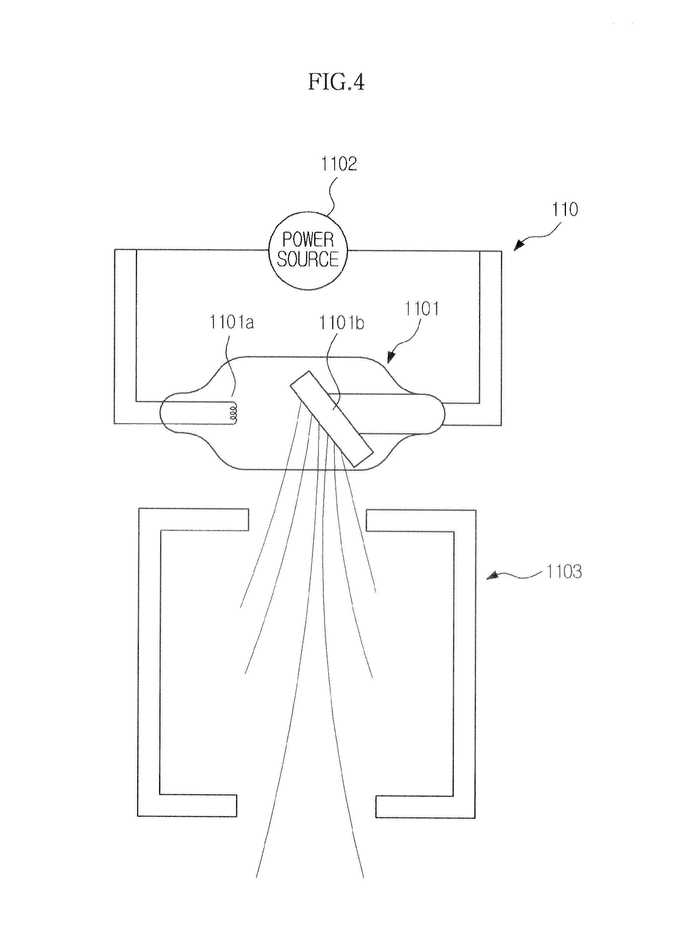

FIG. 4 is a view illustrating an embodiment of the X-ray emitter.

Referring to FIG. 4, the X-ray emitter 110 includes an X-ray tube 1101 that generates X-rays having an energy level corresponding to a voltage applied thereto, and a power source 1102 that is electrically connected to the X-ray tube 1101 to apply a predetermined voltage to the X-ray tube 1101.

If a predetermined voltage is applied from the power source 1102 to the X-ray tube 1101, electrons at a cathode filament 1101a within the X-ray tube 1101 are accelerated toward an anode 1101b according to the applied voltage. When the accelerated electrons collide with the anode 1101b and are rapidly reduced in speed by Coulomb force, X-rays are generated from the anode 1101b based on energy conservation. The generated X-rays are emitted in a predetermined direction, for example, downward of the drawing as exemplarily illustrated in FIG. 4.

The emitted X-rays pass through a collimator 1103 that is located in an X-ray path, for example, at one side of the X-ray tube 1101. The collimator 1103 absorbs and filters X-rays emitted in directions except for a direction selected by the user, thereby ensuring emission of X-rays only in the selected direction. Through use of the collimator 1103, the user may control an X-ray emission direction or X-ray emission range.

The X-ray emitter 110, as exemplarily illustrated in FIGS. 2 and 3, may further include a housing in which the X-ray tube 1101, the power source 1102, or the collimator 1103 is accommodated.

Referring again to FIG. 2, the X-ray imaging apparatus may include the X-ray detection module 12 in the form of a table.

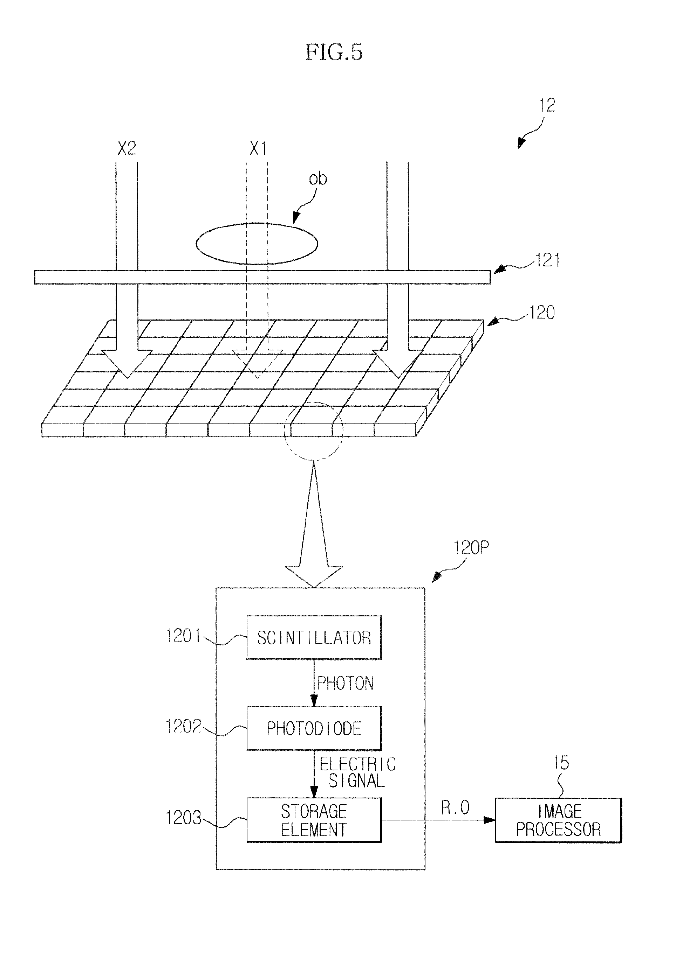

FIG. 5 is a view illustrating an embodiment of the X-ray detection module.

The X-ray detection module 12 includes a cradle 121 on which the subject *ob* may be placed, and the X-ray detector 120 that receives X-rays x1 having passed through the subject *ob* or X-rays x2 directed around the subject *ob.* Here, the X-ray detector 120 may be movably designed.

More specifically, the X-ray detector 120 may be an X-ray detection panel to detect X-rays. The X-ray detection panel may be divided into a plurality of pixels 120p. Each pixel 120p may include a scintillator 1201, a photodiode 1202, and a storage element 1203.

The scintillator 1201 outputs visible photons via light emission upon receiving incident X-rays. The photodiode 1202 receives the visible photons and changes the photons into electric signals, for example, into analog data. The storage element 1203, for example, a capacitor stores the electric signals. The electric signals stored in the storage element 1203 are read out by the image processor 15. The image processor 15 generates an X-ray image using the readout electric signals to assist the user in viewing the interior of the subject *ob*. The X-ray detector 120 may further include a substrate on which the scintillator 1201, the photodiode 1202, and the storage element 1203 as described above are mounted.

Although not illustrated in the drawings, according to embodiments, a collimator may be additionally provided between the subject *ob* and the X-ray detector 120, to filter X-rays scattered while passing through the subject *ob*. In this case, the collimator may be attached to the above-described X-ray detector 120 so as to be moved along with the X-ray detector 120.

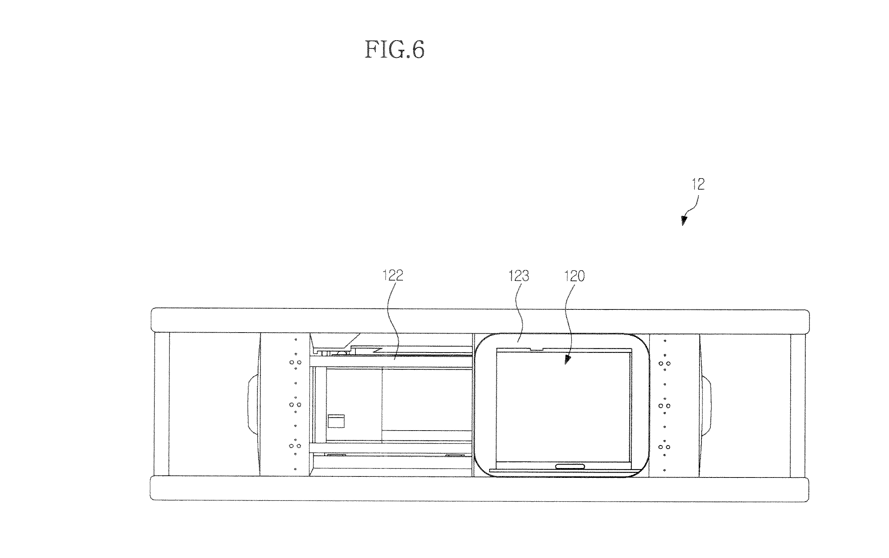

FIG. 6 is a view illustrating one embodiment of the X-ray detection module. The X-ray detection module 12 may further include a rail 122 on which the X-ray detector 120 is placed so as to be moved to a predetermined position. In this case, the X-ray detection module 12 may include, for example, the two-row rail 122. An X-ray detector case 123 is placed on the rail 122. The X-ray detector case 123 may be provided at a lower surface or a lateral surface thereof with canisters. The canisters may come into contact with the rail 122 and may be guided along the rail 122, to ensure that the X-ray detector 120 is stably movable on the rail 122. The X-ray detector 120 may be secured to the X-ray detector case 123. As such, if the X-ray detector case 123 is moved along the rail 122, the X-ray detector 120 secured to the X-ray detector case 123 is also equally moved. Consequently, the X-ray detector 120 may be moved to a predetermined position along the rail 122.

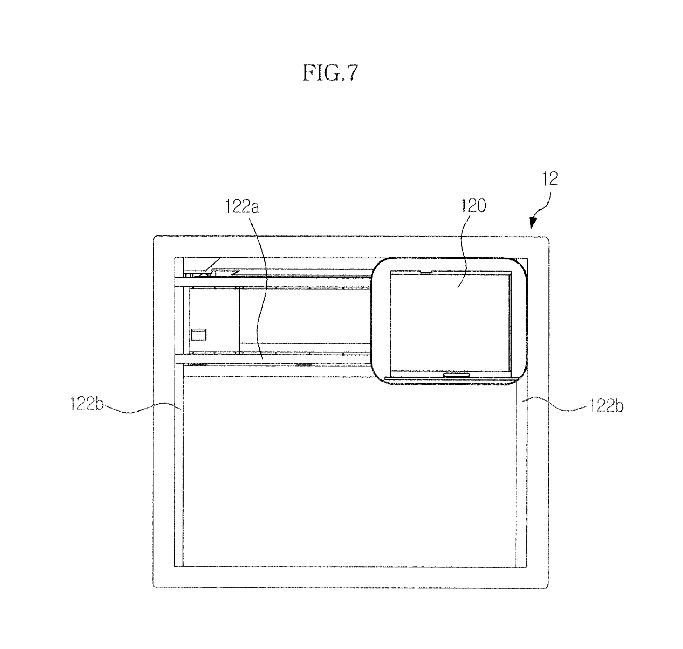

FIG. 7 is a view illustrating another embodiment of the X-ray detection module 12. As exemplarily illustrated in FIG. 7, the X-ray detection module 12 may include a plurality of two-row rails 122a and 122b. If the rail 122 is installed only in a given direction as exemplarily illustrated in FIG. 6, the X-ray detector 120 may be one-dimensionally moved only in the installation direction of the rail 122. The X-ray detection module 12 as exemplarily illustrated in FIG. 7 including the plurality of two-row rails 122a and 122b enables two-dimensional movement of the X-ray detector 120 in the X-axis and the Y-axis.

Although not illustrated in the drawings, according to another embodiment of the X-ray detection module 12, the rail may be replaced with, for example, a robot arm to move the X-ray detector 120. According to another embodiment, the X-ray detector 120 may be moved to a predetermined position by the canisters of the X-ray detector case 123 without the rail, or may be moved to a predetermined position by operation of a pneumatic or hydraulic cylinder connected to the X-ray detector 120 or the X-ray detector case 123. In addition, various other devices to move a position of a designated object may be used to move the X-ray detector 120 within a predetermined range.

Referring again to FIG. 1 B, the controller 13 of the X-ray imaging apparatus 10 may control movements of the X-ray emitter 110 and the X-ray detector 120.

In one embodiment, the controller 13 may acquire or generate information regarding a target position of at least one of the X-ray emitter 110 and the X-ray detector 120, and thereafter may move at least one of the X-ray emitter 110 and the X-ray detector 120 based on the acquired or generated target position information.

The target position information is information regarding a position to which the X-ray emitter 110 or the X-ray detector 120 will be moved according to user operation or preset setting. The target position information, for example, may include predetermined coordinates with regard to a target position to which the X-ray emitter 110 or the X-ray detector 120 will be moved.

To control movements of the X-ray emitter 110 and the X-ray detector 120 based on the target position information, the controller 13 may transmit the acquired or generated target position information regarding the X-ray emitter 110 and the X-ray detector 120 to each of the X-ray emitter 110 and the X-ray detector 120. The X-ray emitter 110 and the X-ray detector 120 are moved to target positions based on the transmitted information. In another example, the controller 13 may acquire or generate target position information regarding each of the X-ray emitter 110 and the X-ray detector 120, and generate movement instructions for the X-ray emitter 110 and the X-ray detector 120 based on the acquired or generated target position information regarding the X-ray emitter 110 and the X-ray detector 120 so as to transmit the instructions to the X-ray emitter 110 and the X-ray detector 120.

The controller 13, as exemplarily illustrated in FIG. 1 B, transmits target position information position or movement instructions to the X-ray emitter 110 and the X-ray detector 120 respectively. In other words, transmission of target position information or movement instructions to the X-ray emitter 110 is performed separately from transmission of target position information or movement instructions to the X-ray detector 120. In this case, generation of target position information or movement instructions with regard to the X-ray emitter 110 may be performed separately from or simultaneously with generation of target position information or movement instructions with regard to the X-ray detector 120.

The above-described controller 13 may be a processor installed in the X-ray emission module 11 or the X-ray detection module 12, or may be a separate information-processing device, such as a computer, or a processor of an image-processing device, which may perform data transmission/reception with the X-ray emission module 11 or the X-ray detection module 12.

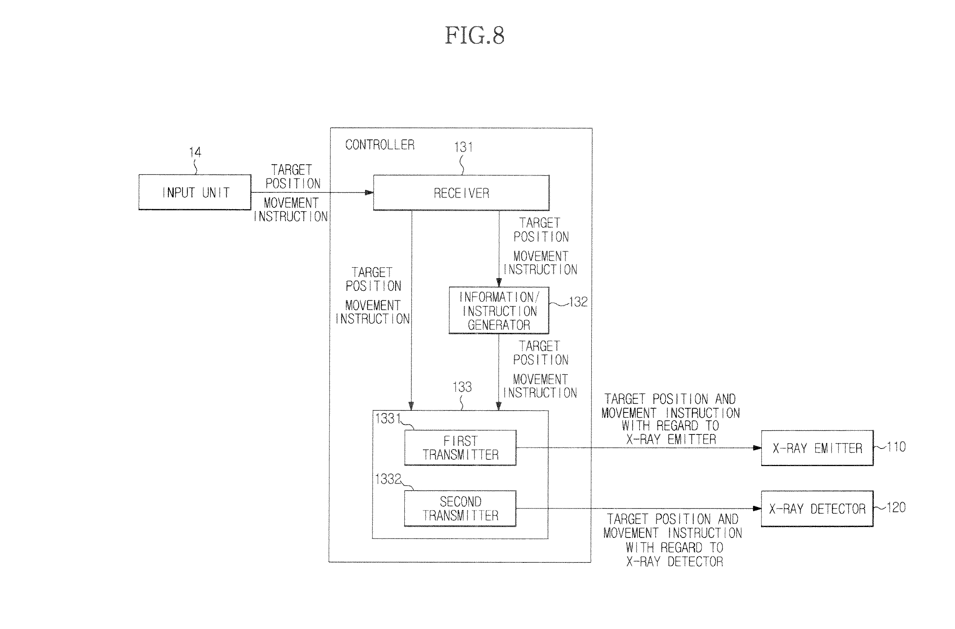

FIG. 8 is a block diagram illustrating an embodiment of the controller. Referring to FIG. 8, the controller 13 includes a receiver 131 that receives information or instructions through the external input unit 14, an information/instruction generator 132 that generates novel information or instructions based on the received information or instructions, and a transmitter 133 that transmits the received information or instructions or the novel information or instructions to an appropriate subject.

The receiver 131 receives information input through the input unit 14, for example, target position information regarding the X-ray emitter 110 or target position information regarding the X-ray detector 120, or a movement instruction for the X-ray emitter 110 or a movement instruction for the X-ray detector 120.

The information/instruction generator 132 generates novel information or instructions based on the received information or instructions. More specifically, the information/instruction generator 132 generates information required to control movements of the X-ray emitter 110 and the X-ray detector 120, for example, target position information or related instructions, for example, movement instructions, and transmits the generated information or instructions to at least one of the X-ray emitter 110 and the X-ray detector 120.

For example, if the user inputs target position information regarding the X-ray emitter 110 as well as target position information regarding the X-ray detector 120 through the input unit 14, the information/instruction generator 132 of the controller 13 generates a plurality of movement instructions based on a plurality of positions input through the receiver 131 as necessary. In other words, the information/instruction generator 132 generates a movement instruction for the X-ray emitter 110 based on target position information regarding the X-ray emitter 110, and generates a movement instruction for the X-ray detector 120 based on target position information regarding the X-ray detector 120.

If the controller 13 receives target position information regarding any one of the X-ray emitter 110 and the X-ray detector 120, for example, target position information regarding the X-ray emitter 110 through the receiver 131, the information/instruction generator 132 may calculate an optimal position to allow the X-ray detector 120 to receive X-rays emitted from the X-ray emitter 110 based on the received target position information regarding the X-ray emitter 110, thereby generating target position information regarding the X-ray detector 120. In addition, as necessary, the information/instruction generator 132 may generate a movement instruction for the X-ray emitter 110 to allow the X-ray emitter 110 to be moved based on the received target position information regarding the X-ray emitter 110, and a movement instruction for the X-ray detector 120 to allow the X-ray detector 120 to be moved based on the received target position information regarding the X-ray detector 120.

On the contrary, If the controller 13 receives target position information regarding the X-ray detector 120, the information/instruction generator 132 may generate target position information regarding the X-ray emitter 110, and generate an appropriate movement instruction for the X-ray emitter 110 or the X-ray detector 120 to allow the X-ray emitter 110 or the X-ray detector 120 to be moved based on the received or generated target position information.

The transmitter 133 transmits target position information regarding the X-ray emitter 110 and target position information regarding the X-ray detector 120, or a movement instruction for the X-ray emitter 110 and a movement instruction for the X-ray detector 120 to the X-ray emitter 110 and the X-ray detector 120. In this case, target position information regarding the X-ray emitter 110 and target position information regarding the X-ray detector 120 may be information received by the receiver 131, or may be information generated by the information/instruction generator 132. In addition, a movement instruction for the X-ray emitter 110 or a movement instruction for the X-ray detector 120 may be instructions received by the receiver 131, or may be instructions generated by the information/instruction generator 132.

In particular, the transmitter 133 may include a first transmitter 1331 and a second transmitter 1332 as exemplarily illustrated in FIG. 8. The first transmitter 1331 transmits target position information and a movement instruction with regard to the X-ray emitter 110, and the second transmitter 1332 transmits target position information and a movement instruction with regard to the X-ray detector 120. In this case, the first transmitter 1331 and the second transmitter 1332 may independently perform the above-described functions. Through the first transmitter 1331 and the second transmitter 1332, the transmitter 132 may transmit target position information or movement instructions to both the X-ray emitter 110 and the X-ray detector 120 respectively.

Accordingly, the X-ray emitter 110 and the X-ray detector 120 may independently receive target position information or movement instructions at the same time or at different times. In this way, the user may individually control operations of the X-ray emitter 110 and the X-ray detector 120, for example, movements thereof to respective target positions, and as necessary may independently move the X-ray emitter 110 and the X-ray detector 120.

As such, it may be unnecessary to first move the X-ray emitter 110 and thereafter move the X-ray detector 120 based on an actual moved position of the X-ray emitter 110, or conversely to first move the X-ray detector 120 and thereafter move the X-ray emitter 110 based on the movement result.

If the X-ray emitter 110 receives target position information regarding the X-ray emitter 110 and the X-ray detector 120 receives target position information regarding the X-ray detector 120 from the controller 13, the X-ray emitter 110 is moved in response to a movement instruction for the X-ray emitter 110 that is generated by a separate processor equipped in the X-ray emitter 110 based on the received target position information regarding the X-ray emitter 110. In this case, the X-ray emitter 110 will be moved to a position corresponding to the received target position information regarding the X-ray emitter 110. Likewise, the X-ray detector 120 is moved to a position corresponding to the received target position information regarding the X-ray detector 120 in response to a control instruction that is generated by a separate processor equipped in the X-ray detector 120 based on the target position information regarding the X-ray detector 120.

If the X-ray emitter 110 receives a movement instruction for the X-ray emitter 110 and the X-ray detector 120 receives a movement instruction for the X-ray detector 120 from the controller 13, the X-ray emitter 110 and the X-ray detector 120 are moved to respective target positions thereof in response to the received movement instructions from the controller 13. In addition, the X-ray emitter 110 may receive a movement instruction for the X-ray emitter 110 from the controller 13 and the X-ray detector 120 may receive target position information regarding the X-ray detector 120, and the contrary case may also be possible.

According to embodiments, the controller 13 may transmit target position information or a movement instruction to the X-ray emitter 110 or the X-ray detector 120, and may receive information regarding a current position of the X-ray emitter 110 or the X-ray detector 120 from the X-ray emitter 110 or the X-ray detector 120 after the X-ray emitter 110 or the X-ray detector 120 is moved based on the transmitted target position information or movement instruction.

The controller 13 may verify whether or not the X-ray emitter 110 or the X-ray detector 120 is moved to an accurate position by comparing the information received from the X-ray emitter 110 or the X-ray detector 120 with the target position information or movement instruction transmitted to the X-ray emitter 110 or the X-ray detector 120. If there are errors between the current position, target position information, and movement instruction of the X-ray emitter 110 or the X-ray detector 120, the controller 13 may transmit novel target position information or a novel movement instruction to the X-ray emitter 110 or the X-ray detector 120 to compensate for the errors.

As exemplarily illustrated in FIGS. 1B and 5, the X-ray imaging apparatus 10 may include the input unit 14 to allow the user to input information or instructions. The input unit 14 receives, from the user, at least one of target position information on the X-ray emitter 110 with regard to a position to which the X-ray emitter 110 will be moved, and target position information on the X-ray detector 120 with regard to a position to which the X-ray detector 120 will be moved.

In this case, the input unit 14 may be any one or combinations of at least two ones of a joystick, a keyboard, a keypad, a paddle, a handle, a touchscreen, a track ball, a mouse, and a tablet PC. In one example, the input unit 14 may be formed of only at least one keyboard, at least one keypad, or at least one joystick. In another example, the input unit 14 may be formed of a combination of a joystick and a keyboard, or a combination of a touchscreen and a track ball. If the input unit 14 is a tablet PC, the user may input information or instructions using fingers or a touch pen.

The input unit 14 may transmit information or instructions input by the user to the controller 13 via wired communication using cables, or via wireless radio, light, sound, or ultrasound communication. Here, wireless radio communication includes digital mobile communication, and wireless light communication includes wireless infrared communication that transmits data or instructions using infrared light. In other words, the input unit 14 may remotely control the X-ray imaging apparatus by transmitting data, for example, movement position information regarding the X-ray emitter 110 or the X-ray detector 120 to the controller 13.

Referring to FIG. 1 B, the X-ray imaging apparatus 10 may further include the image processor 15 that generates an X-ray image based on electric signals stored in the storage element 1203 of the X-ray detector 120, or that corrects the generated X-ray image by performing predetermined image-processing on the generated X-ray image. According to embodiments, the image processor 15 may be a processor equipped in the X-ray detection module 12, or may be a processor of an external image-processing device that may perform data transmission/reception with the X-ray detection module 12 via a wired or wireless network.

The X-ray image acquired by the image processor 15 is displayed to the user through the display unit 16. The display unit 16 may be a display device, such as a monitor, etc., which is equipped in the X-ray imaging apparatus 10, or connected to an external image-processing device through cables.

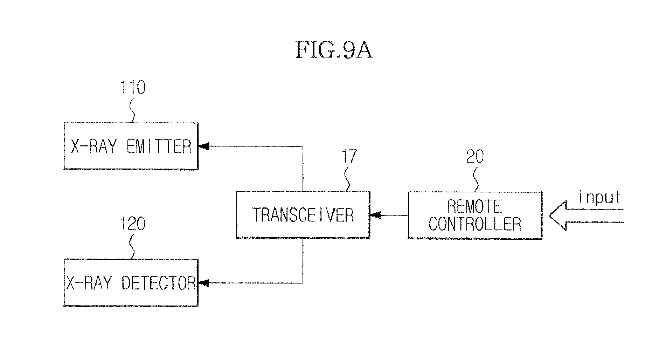

FIG. 9A is a view illustrating another embodiment of a system to control the X-ray emitter and the X-ray detector in the X-ray imaging apparatus.

According to the present embodiment as exemplarily illustrated in FIG. 9A, the X-ray emitter 110 and the X-ray detector 120 of the X-ray imaging apparatus 10 receive information or instructions from a remote controller 20 and are controlled by the remote controller 20 so as to be moved or to emit X-rays according to the received information or instructions. In this case, the X-ray emitter 110 and the X-ray detector 120, for example, may receive information or instructions through a transceiver 17 that may perform data transmission/reception with the remote controller 20.

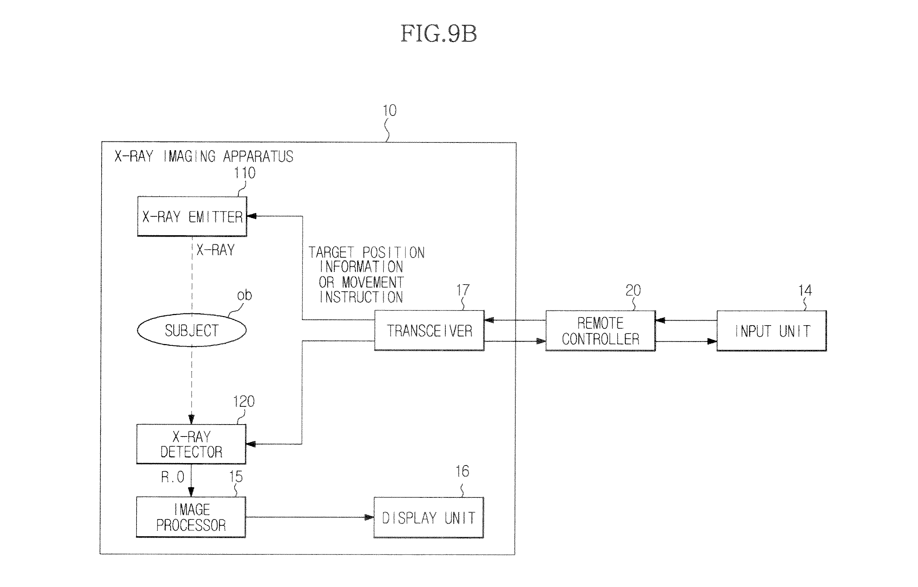

FIG. 9B is a view illustrating a configuration of the X-ray imaging apparatus according to another embodiment.

According to the present embodiment as exemplarily illustrated in FIG. 9B, the X-ray imaging apparatus may include the X-ray emitter 110, the X-ray detector 120, the image processor 15, the display unit 16, and the transceiver 17.

The X-ray emitter 110 may generate X-rays and emit the generated X-rays to the subject as described above with reference to FIGS. 1 B to 4, and may be moved to a predetermined position to thereby emit X-rays as exemplarily illustrated in FIG. 3.

The X-ray detector 120 may receive X-rays emitted from the X-ray emitter 110 and change the received X-rays into electric signals as described above with reference to FIGS. 5 to 7, and may be moved to a predetermined position to thereby receive X-rays as exemplarily illustrated in FIGS. 6 and 7.

The image processor 15 generates an X-ray image based on the electric signals acquired by the X-ray detector 120, and corrects an X-ray image by performing predetermined image-processing on the generated X-ray image.

The display unit 16 displays the X-ray image generated or corrected by the image processor 15 to the user. According to embodiments, the display unit 16 may be equipped in the X-ray imaging apparatus, or may be included in a monitor device connected to an external image-processing device.

According to one embodiment, the X-ray imaging apparatus 10 may further include the transceiver 17 that receives target position information or control instructions with regard to the X-ray emitter 110, for example, movement instructions for the X-ray emitter 110, from the remote controller 20.

The transceiver 17 may transmit control instructions or data received from the remote controller 20 to the X-ray emitter 110 and the X-ray detector 120 respectively, to allow the X-ray emitter 110 and the X-ray detector 120 to be driven according to information or control instructions transmitted from the remote controller 20.

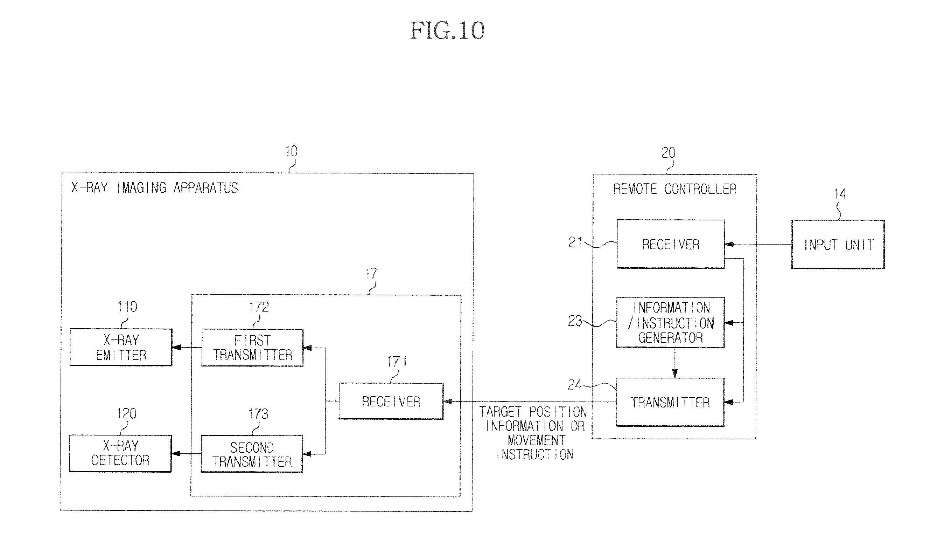

FIGS. 10 to 12 are views explaining an embodiment of the remote controller and the X-ray imaging apparatus.

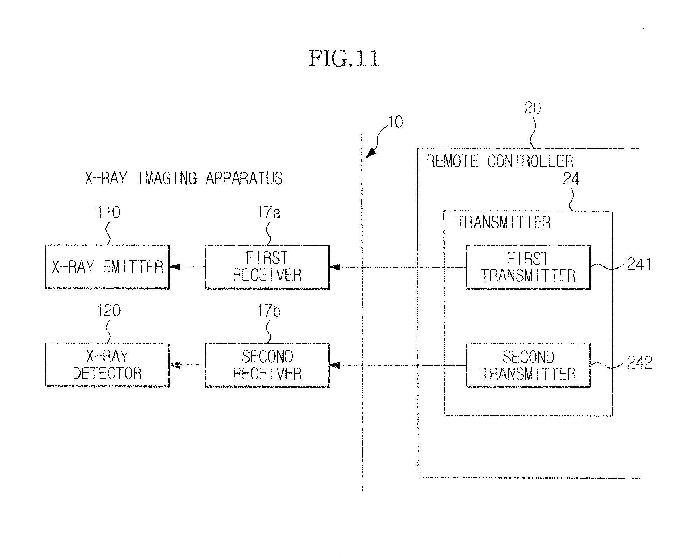

Referring to FIGS. 10 and 11, the X-ray imaging apparatus 10 may receive information or control instructions from the remote controller 20, for example, target position information or movement instructions regarding the X-ray emitter 110 or the X-ray detector 120, and may be controlled according to the received information or control instructions.

According to an embodiment as exemplarily illustrated in FIG. 10, the remote controller 20 may include a receiver 21 that receives data or control instructions transmitted from the external input unit 14, an information/instruction generator 23 that generates novel data or control instructions based on the data or control instructions received by the receiver 21, and a transmitter 24 that transmits the data or control instructions received by the receiver 21 or generated by the information/instruction generator 23 to a receiver 171 of the X-ray imaging apparatus.

The input unit 14 may allow the X-ray imaging apparatus to be controlled upon receiving predetermined instructions from the user. More specifically, the input unit 14 may receive, from the user, at least one of target position information on the X-ray emitter 110 with regard to a position to which the X-ray emitter 110 will be moved, and target position information on the X-ray detector 120 with regard to a position to which the X-ray detector 120 will be moved. As necessary, the input unit 14 may generate a movement control instruction for the X-ray emitter 110 or the X-ray detector 120 based on target position information regarding the X-ray emitter 110 or the X-ray detector 120 input by the user.

According to the embodiment, the input unit 14, as exemplarily illustrated in FIG. 10, may be isolated from the remote controller 20, and may transmit the control instructions or data input by the user to the remote controller 20 via wired communication using cables, or via wireless radio, light, sound, or ultrasound communication. Here, the input unit 14, for example, may be any one or combinations of at least two ones of a joystick, a keyboard, a keypad, a paddle, a handle, a touchscreen, a track ball, a mouse, and a tablet PC.

The information/instruction generator 23 of the remote controller 20 generates novel information or instructions, for example, target position information or movement instructions with regard to the X-ray emitter 110 or the X-ray detector 120, based on data or control instructions input through the input unit 14.

The transmitter 24 may transmit data or control instructions, input through the input unit 14 or generated by the information/instruction generator 23, for example, target position information or movement instructions with regard to the X-ray emitter 110 or the X-ray detector 120, to the transceiver 17 of the X-ray imaging apparatus 10, thereby allowing at least one of the X-ray emitter 110 and the X-ray generator 120 to be controlled. In this case, for transmission of information or control instructions between the transmitter 24 and the transceiver 17 of the X-ray imaging apparatus 10, cables, radio, light, sound, or ultrasound may be adopted as transmission media.

As necessary, in addition to data or control instructions, the transmitter 24 may transmit identification code, which identifies whether the data or control instructions refer to any one of the X-ray emitter 110 and the X-ray detector 120, to the transceiver 17.

If the transmitter 24 transmits data or control instructions to the transceiver 17 of the X-ray imaging apparatus 10, the transceiver 17 of the X-ray imaging apparatus 10 receives data or control instructions transmitted from the transmitter 24 of the remote controller 20, for example, target position information or movement instructions with regard to the X-ray emitter 110 or the X-ray detector 120, through the receiver 171.

In this case, as exemplarily illustrated in FIG. 10, the transceiver 17 of the X-ray imaging apparatus 10 judges whether data or control instructions received through the receiver 171 are data or control instructions with regard to any one of the X-ray emitter 110 and the X-ray detector 120, and transmits the judged result to each device. In this case, for example, the aforementioned identification code may be used to judge whether data or control instructions with regard to any device are given.

The transceiver 17 may include a first transmitter 172 and a second transmitter 173. For example, the first transmitter 172 transmits data or instructions, for example, target position information or movement instructions with regard to the X-ray emitter 110, to the X-ray emitter 110, and the second transmitter 173 transmits data or instructions, for example, target position information or movement instructions with regard to the X-ray detector 120, to the X-ray detector 120.

The first transmitter 172 and the second transmitter 173 may be independently operated to transmit data or instructions to each device. Thereby, the X-ray emitter 110 and the X-ray detector 120 may be individually controlled at the same time or at different times, and as necessary may be operated so as to be independently moved.

According to another embodiment, the transmitter 24 of the remote controller 20, as exemplarily illustrated in FIG. 11, may include a first transmitter 241 and a second transmitter 242, which transmit data or information individually according to identification code. In this case, X-ray imaging apparatus may include a first receiver 17a and a second receiver 17b so as to correspond to the respective transmitters 241 and 242.

For example, the first transmitter 241 may transmit target position information or a movement instruction with regard to the X-ray emitter 110 to the first receiver 17a of the remote controller 20, and the second transmitter 242 may transmit target position information or a movement instruction with regard to the X-ray detector 120 to the second receiver 17b of the remote controller 20. Then, the first receiver 17a and the second receiver 17b transmit the received target position information or movement instructions with regard to the X-ray emitter 110 and the X-ray detector 120 to the X-ray emitter 110 and the X-ray detector 120 respectively, thereby allowing the X-ray emitter 110 and the X-ray detector 120 to be moved to predetermined positions.

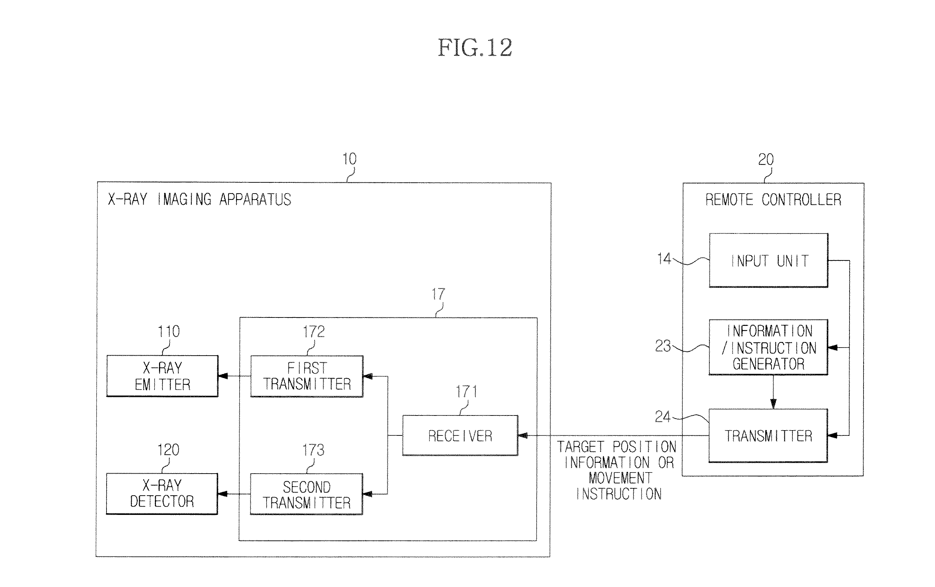

According to another embodiment as exemplarily illustrated in FIG. 12, the remote controller 20 may include the input unit 14 that receives data or control instructions from the user, the information/instruction generator 23 that generates novel data or control instructions based on the data or control instructions input through the input unit 14, and the transmitter 24 that transmits the data or control instructions input through the input unit 14 or generated by the information/instruction generator 23 to the receiver 171 of the X-ray imaging apparatus. In other words, the above-described input unit 14, for example, any one of a joystick, a keyboard, a keypad, a paddle, a handle, a touchscreen, a track ball, a mouse, and a tablet PC, by which the user inputs a predetermined instruction, may be mounted in the remote controller 20.

If the user inputs data or instructions, for example, target position information regarding the X-ray emitter 110 or the X-ray detector 120 using the input unit 14 mounted in the remote controller 20, the input data may be directly transmitted to the transceiver 17 of the X-ray imaging apparatus 10. In addition, based on the data input through the input unit 14, for example, control instructions related to the data may be generated by the information/instruction generator 23 of the remote controller 20.

For example, the information/instruction generator 23 as exemplarily illustrated in FIG. 12 may generate a control instruction that commands the X-ray emitter 110 or the X-ray detector 120 to be moved to a target position based on information regarding the target position. The generated control instruction is transmitted to the transceiver 17 of the X-ray imaging apparatus 10 and consequently transmitted to the X-ray emitter 110 or the X-ray detector 120.

Moreover, the information/instruction generator 23 may generate novel data or control instructions based on data input by the user as necessary. For example, the information/instruction generator 23 may generate target position information regarding the X-ray detector 120 based on target position information regarding the X-ray emitter 110 input through the input unit 14. Of course, the contrary case may also be possible. Then, the information/instruction generator 23 may generate control instructions for the X-ray emitter 110 and the X-ray detector 120, for example, movement instructions for the X-ray emitter 110 and the X-ray detector 120 based on the input data, for example, target position information regarding the X-ray emitter 110 as well as the generated novel data, for example, target position information regarding the X-ray detector 120.

The generated novel data or control instructions are transmitted to the transceiver 17 of the X-ray imaging apparatus 10. The transceiver 17 transmits the received data or control instructions, for example, target position information or movement instructions with regard to the X-ray emitter 110, to the X-ray emitter 110, and target position information or movement instructions with regard to the X-ray detector 120, to the X-ray detector 120.

Although the remote controller 20 as described above with reference to FIGS. 9A to 12 may be a device that is separately fabricated to operate the X-ray imaging apparatus according to one embodiment, the remote controller 20 may be any one of various mobile devices, such as a mobile phone, a smart-phone, a Personal Digital Assistant (PDA), or a tablet PC according to embodiments. In addition, the remote controller 20 may be any information-processing device, such as a personal computer, or may be any one of various terminal devices, such as an infrared remote controller. As necessary, the remote controller may be a combination of at least two devices among the above-enumerated devices.

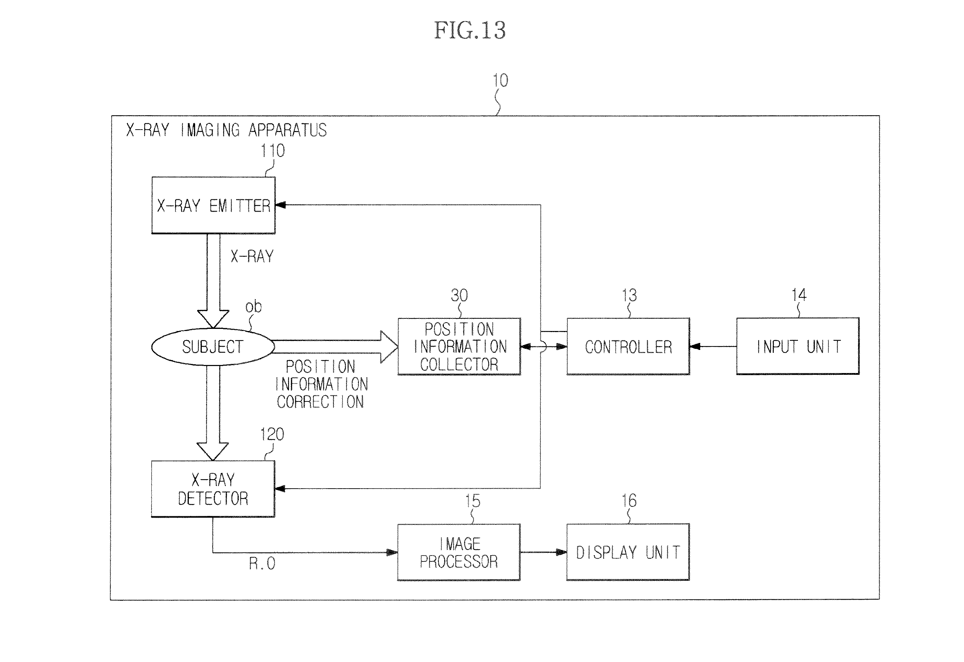

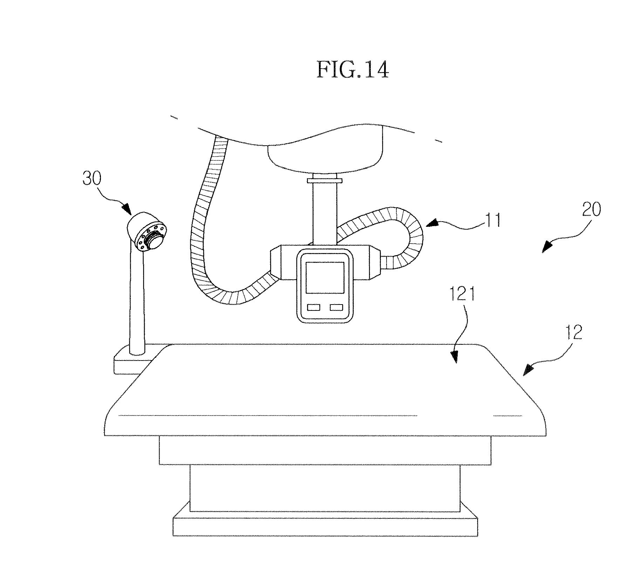

FIGS. 13 and 14 are views of an X-ray imaging apparatus equipped with a position information collector.

The X-ray imaging apparatus 10, as exemplarily illustrated in FIGS. 13 and 14, may further include a position information collector 30. The position information collector 30 collects position information regarding the subject *ob* placed on the cradle 121 of the X-ray emission module 12, for example, 3D coordinate information regarding the subject *ob*. The position information collector 30, as exemplarily illustrated in FIG. 14, is located at a predetermined position of the X-ray imaging apparatus 10, for example, at the edge of the X-ray detection module 12 or at a lower end of the X-ray emission module 11.

The position information collector 30 may be, for example, a range sensor, a 3D camera, a 3D depth camera, a 3D color/depth camera, a stereo camera, an infrared camera, and a Position Sensitive Device (PSD). In addition, the position information collector 20 may be a combination of at least two devices among the above-enumerated devices. In this case, position information regarding the subject *ob* is collected using a plurality of sensors.

The position information collector 30 may acquire position information regarding the subject *ob*, for example, 3D coordinates of the subject *ob* by emitting light to the subject *ob*, receiving the light reflected from the subject *ob*, and measuring a distance between a camera and the subject *ob* based on a time difference between light reception and light transmission.

The controller 13, as described above, receives position information regarding the subject *ob* collected by the position information collector 30, and generates information on target positions, to which the X-ray emitter 110 and the X-ray detector 120 will be moved, i.e. target position information regarding the X-ray emitter 110 and the X-ray detector 120, or movement instructions for the X-ray emitter 110 and the X-ray detector 120, based on the collected position information.

When generating target position information or movement instructions with regard to the X-ray emitter 110 and the X-ray detector 120, the controller 13 may generate target position information by calculating optimal positions to which the X-ray emitter 110 and the X-ray detector 120 will be moved, or may generate movement instructions based on the generated target position information, to ensure that the X-ray emitter 110 and the X-ray detector 120 appropriately capture an image of the subject *ob*. In this case, the controller 130, as described above, may generate target position information or movement instructions with regard to the X-ray emitter 110 or the X-ray detector 120.

In addition, the controller 13, as exemplarily illustrated in FIG. 1A, may transmit target position information or a movement instruction with regard to the X-ray emitter 110 to the X-ray detector 120, and may transmit target position information or a movement instruction with regard to the X-ray detector 120 to the X-ray emitter 110. In this case, transmission of information to the X-ray emitter 110 and transmission of information to the X-ray detector 120 may be independently performed.

As necessary, the controller 13 may receive data or instructions from the user, and transmit the input data or instructions to the X-ray emitter 110 and the X-ray detector 120. In addition, the controller 13 may generate control instructions to control the position information collector 30 according to embodiments.

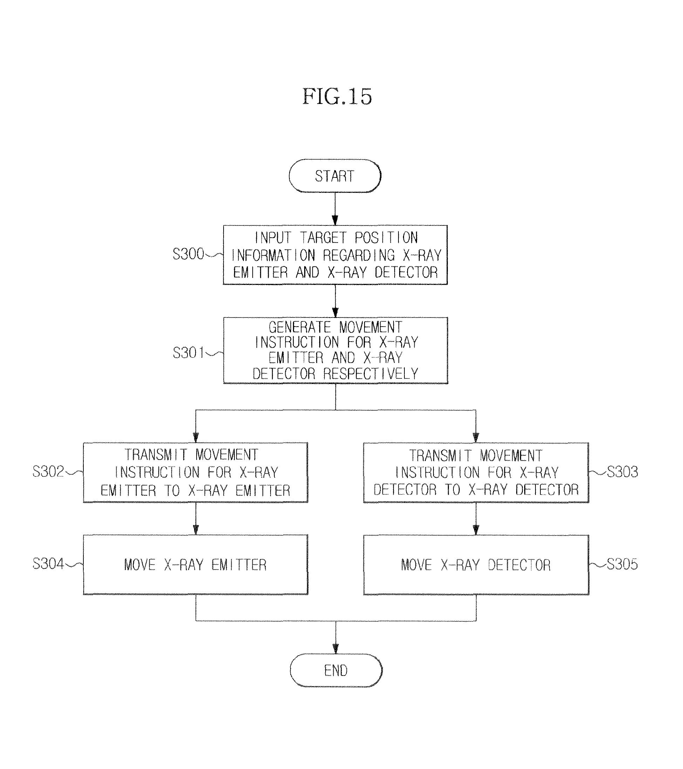

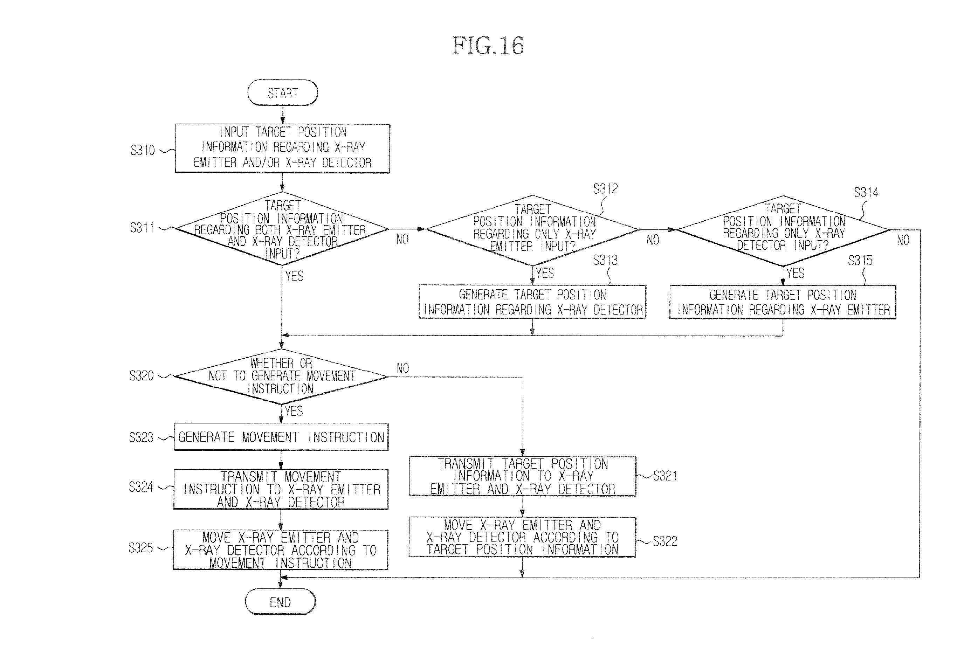

FIGS. 15 and 16 are flowcharts illustrating a control method for the X-ray imaging apparatus.

Referring to FIG. 15, according to one embodiment of the control method for the X-ray imaging apparatus, first, the X-ray imaging apparatus 10 receives target position information regarding the X-ray emitter 110 and target position information regarding the X-ray detector 120 through the input unit 14 or the position information collector 30 (S300).

The X-ray imaging apparatus 10 generates movement instructions for the X-ray emitter 110 and the X-ray detector 120 respectively based on the input target position information regarding the X-ray emitter 110 and the X-ray detector 120 (S301).

Thereafter, the generated movement instruction for the X-ray emitter 110 is transmitted to the X-ray emitter 110 and apart from this, the generated movement instruction for the X-ray detector 120 is transmitted to the X-ray detector 120 (S302 and S303).

The X-ray emitter 110 and the X-ray detector 120 are independently moved according to the transmitted movement instructions (S304 and S305).

According to another embodiment of the control method for the X-ray imaging apparatus, as exemplarily illustrated in FIG. 16, first, the X-ray imaging apparatus 10 receives target position information regarding at least one of the X-ray emitter 110 and the X-ray detector 120 (S310). That is, the X-ray imaging apparatus 10 may receive target position information regarding any one of the X-ray emitter 110 and the X-ray detector 120, or may receive target position information regarding both the X-ray emitter 110 and the X-ray detector 120.

Here, if target position information regarding both the X-ray emitter 110 and the X-ray detector 120 is input (S311), it is judged whether or not to generate movement instructions for the X-ray generator 110 and the X-ray detector 120 based on the target position information regarding both the X-ray emitter 110 and the X-ray detector 120 (S320). If generation of no movement instructions is judged (No in Operation S320), the target position information regarding the X-ray emitter 110 and the target position information regarding the X-ray detector 120 are transmitted respectively to the X-ray emitter 110 and the X-ray detector 120 (S321). Thereby, the X-ray emitter 110 and the X-ray detector 120 are moved respectively according to the transmitted target position information.

If generation of movement instructions for the X-ray emitter 110 and the X-ray detector 120 is judged (Yes in Operation S320), the X-ray imaging apparatus 10 generates a movement instruction for the X-ray emitter 110 and a movement instruction for the X-ray detector 120 (S323) via, for example, the controller 13, and transmits the generated movement instructions to the X-ray emitter 110 and the X-ray detector 120 (S324). The X-ray emitter 110 and the X-ray detector 120 are controlled so as to be moved according to the generated movement instructions for the X-ray emitter 110 and the X-ray detector 120 (S325).

If the input target position information includes only target position information regarding the X-ray emitter 110 (S312), target position information regarding the X-ray detector 120 is generated based on the target position information regarding the X-ray emitter 110 (S313).

If the input target position information includes only target position information regarding the X-ray detector 120 (S314), target position information regarding the X-ray emitter 110 is generated based on the target position information regarding the X-ray detector 120 (S315).

Thereafter, as described above, according to whether or not to generate movement instructions for the X-ray emitter 110 and the X-ray detector 120 (S320), target position information regarding the X-ray emitter 110 and target position information regarding the X-ray detector 120 are transmitted respectively to the X-ray emitter 110 and the X-ray detector 120 (S321), or a movement instruction for the X-ray emitter 110 and a movement instruction for the X-ray detector 120 are generated (S323). Thereafter, the generated movement instructions are transmitted respectively to the X-ray emitter 110 and the X-ray detector 120 (S324).

The X-ray emitter 110 and the X-ray detector 120 are moved based on the transmitted target position information or movement instructions (S322 and S325).

As is apparent from the above description, according to an X-ray imaging apparatus, a remote controller for the X-ray imaging apparatus, and a control method for the X-ray imaging apparatus, it may be possible to control movements of both an X-ray emitter that emits X-rays to a subject and an X-ray detector that receives the emitted X-rays.

Further, it may be possible to transmit information regarding target positions of the X-ray emitter and the X-ray detector as well as control instructions that command movement to particular positions to both the X-ray emitter and the X-ray detector.

Furthermore, it may be possible to remotely control movements of both the X-ray emitter and the X-ray detector.

In addition, X-ray imaging may be performed at an optimal position by moving the X-ray emitter and the X-ray detector to the optimal position, which enables acquisition of an X-ray image at an accurate view via implementation.

Although the embodiments of the present invention have been shown and described, it would be appreciated by those skilled in the art that changes may be made in these embodiments without departing from the scope of the invention which is defined in the claims.

## Claims

1. An X-ray imaging apparatus comprising:
a movable X-ray emitter to emit X-rays to a subject;
a movable X-ray detector to detect X-rays emitted from the X-ray emitter;
an input unit to receive target position information regarding at least one of the X-ray emitter and the X-ray detector; and
a controller to control movements of the X-ray emitter and the X-ray detector according to the input target position information.

2. The apparatus according to claim 1, wherein the input unit receives target position information regarding the X-ray emitter and target position information regarding the X-ray detector respectively, and
wherein the controller controls movements of the X-ray emitter and the X-ray detector according to the respective input target position information.

3. The apparatus according to claim 1 or 2, wherein the controller transmits the target position information to at least one of the X-ray emitter and the X-ray detector to control movements of the X-ray emitter and the X-ray detector.

4. The apparatus according to one of claims 1 to 3, wherein the controller generates movement instructions for the X-ray emitter and the X-ray detector based on the target position information regarding the X-ray emitter and the X-ray detector to control movements of the X-ray emitter and the X-ray detector.

5. The apparatus according to one of claims 1 to 4, wherein the controller transmits the target position information or movement instructions with regard to the X-ray emitter or the X-ray detector using a wired or wireless communication network.

6. The apparatus according to one of claims 1 to 5, wherein the input unit transmits the input target position information regarding at least one of the X-ray emitter and the X-ray detector via wired communication using cables, or radio, light, sound, or ultrasound wireless communication.

7. The apparatus according to one of claims 1 to 6, wherein the input unit includes at least one of a joystick, a keyboard, a keypad, a handle, a touchscreen, a track ball, a mouse, and a tablet PC.

8. The apparatus according to one of claims 1 to 7, wherein the controller calculates a target position of the X-ray detector using the target position information regarding the X-ray emitter input through the input unit, or calculates a target position of the X-ray emitter using the target position information regarding the X-ray detector input through the input unit.

9. The apparatus according to one of claims 1 to 8, wherein the controller generates a movement instruction for the X-ray emitter or the X-ray detector using the target position information regarding the X-ray emitter or the X-ray detector input through the input unit.

10. The apparatus according to one of claims 1 to 9, further comprising a position information collector to sense the subject and collect position information regarding the subject, and/or
wherein the controller generates target position information regarding the X-ray emitter or target position information regarding the X-ray detector based on the position information regarding the subject collected by the position information collector, and/or
wherein the controller generates a movement instruction for the X-ray emitter or the X-ray detector based on the generated target position information regarding the X-ray emitter or the generated target position information regarding the X-ray detector.

11. The apparatus according to one of claims 1 to 10, wherein the controller receives the target position information from a remote controller, and/or
wherein the remote controller transmits the input target position information regarding at least one of the X-ray emitter and the X-ray detector via wired communication using cables, or radio, light, sound, or ultrasound wireless communication, and/or
wherein the remote controller includes at least one of a mobile phone, a smart-phone, a Personal Digital Assistant, PDA, a tablet PC, a personal computer, and an infrared remote controller, and/or
wherein the remote controller calculates target position information regarding the X-ray detector or the X-ray emitter based on the target position information regarding the X-ray emitter or the X-ray detector input through the input unit, or generates a movement instruction for the X-ray emitter or the X-ray detector based on the target position information regarding the X-ray emitter or the X-ray detector input through the input unit.

12. A control method for an X-ray imaging apparatus comprising a movable X-ray to emit X-rays to a subject and a movable X-ray detector to detect X-rays having passed through the subject, the control method comprising:
receiving at least one of target position information regarding the X-ray emitter and target position information regarding the X-ray detector; and
moving the X-ray emitter and the X-ray detector according to the target position information.

13. The control method according to claim 12, wherein the reception of the target position information includes receiving the target position information regarding the X-ray emitter and the target position information regarding the X-ray detector respectively, and
wherein the movement of the X-ray emitter and the X-ray detector includes moving the X-ray emitter and the X-ray detector according to the respective input target position information.

14. The control method according to claim 12 or 13, wherein the movement of the X-ray emitter and the X-ray detector includes:
generating movement instructions for the X-ray emitter and the X-ray detector based on the target position information regarding the X-ray emitter and the X-ray detector;
transmitting the movement instruction for the X-ray emitter to the X-ray emitter, and apart from this, transmitting the movement instruction for the X-ray detector to the X-ray detector; and
moving the X-ray emitter and the X-ray detector according to the transmitted movement instructions.

15. The control method according to one of claims 12 to 14, wherein the movement of the X-ray emitter and the X-ray detector includes:
determining target position information regarding the X-ray detector based on the target position information regarding the X-ray emitter if the input target position information is the target position information regarding the X-ray emitter, and determining target position information regarding the X-ray emitter based on the target position information regarding the X-ray detector if the input target position information is the target position information regarding the X-ray detector;
transmitting a movement instruction for the X-ray emitter to the X-ray emitter and transmitting a movement instruction for the X-ray detector to the X-ray detector; and
moving the X-ray emitter and the X-ray detector based on the target position information regarding the X-ray emitter and the target position information regarding the X-ray detector.
